# EUROPÄISCHE PATENTSCHRIFT

(11) **EP 1 894 511 B1**
(45) Veröffentlichungstag und Bekanntmachung des Hinweises auf die Patenterteilung: **11.11.2009**
(21) Anmeldenummer: 07016514.7
(22) Anmeldetag: 23.08.2007
(51) Int. Cl.: A61B 1/00

(54) **Stroboskopmodul mit einer Kupplung**
Stroboscope module with a coupler
Module de stroboscope doté d'un engrenage

(30) Priorität: 30.08.2006 DE 102006041950
(43) Veröffentlichungstag der Anmeldung: 05.03.2008
(73) Patentinhaber: Karl Storz GmbH & Co. KG, 78532 Tuttlingen (DE)
(72) Erfinder: Irion, Klaus, 78576 Emmingen-Liptingen (DE); Rebholz, Clemens, 88690 Uhldingen-Mühlhofen (DE); Hensler, Fritz, 78579 Neuhausen (DE); Schmid, Robert, 78549 Spaichingen (DE)
(74) Vertreter: Weller, Wolfgang

(56) Entgegenhaltungen:
- WO-A-01/89598
- DE-A1-102004 052 847
- US-A- 5 643 251

## Beschreibung

Die vorliegende Erfindung betrifft eine Kupplung für ein Stroboskopmodul, die eine Hülse und einen gegen die Hülse verschiebbaren Sockel aufweist, wobei zwischen der Hülse und dem Sockel eine elastisch verformbare Manschette angeordnet ist, in die der Lichtanschluss des Endoskops in einer ersten Stellung einführbar ist, wobei die Hülse und der Sockel in einer zweiten Stellung die Manschette komprimieren, so dass sich der Innendurchmesser der Manschette verkleinert, wobei die Hülse und der Sockel in der zweiten Stellung verriegelbar sind, und wobei die Kupplung ferner einen Lichtleiter aufweist, der beim Anschluss des Stroboskopmoduls an das Endoskop Licht aus dem Stroboskopmodul in den Lichtanschlusses einkoppelt. Die Erfindung betrifft ferner ein Stroboskopmodul mit einer Kupplung.

Stroboskope werden im Zusammenhang mit Endoskopen in der Medizin zur Untersuchung des Kehlkopfes eingesetzt. Das Stroboskop dient dabei dazu, von den während der Tonerzeugung schwingenden Stimmbändern ein stehendes Bild zu erzeugen.

Zu solchen Untersuchungen ist z.B. aus der DE 10 2004 052 847 A1 ein Stroboskopmodul bekannt, das eine stroboskopische Lichtquelle, ein Mikrofon zur Aufnahme von vom Patienten erzeugten Tönen sowie eine Stromquelle, eine Steuereinheit zur Steuerung der stroboskopischen Lichtquelle anhand der von dem Mikrofon aufgenommenen Frequenzen und eine Ausgabeeinheit zur Ausgabe der Frequenzen der vom Patienten erzeugten Töne in einem Modul vereinigt. Die hierbei verwendete Kupplung zum Anschließen des Stroboskopmoduls an einem Lichtanschluss eines Endoskops wird in diesem Dokument lediglich als mechanische Kupplung bezeichnet.

Bei der Entwicklung von Kupplungen zum Anschließen eines Stroboskopmoduls an den Lichtanschluss eines Endoskops hat sich herausgestellt, dass Schraubverbindungen häufig nachteilig sind, da auch ein nur geringes Verdrehen des Moduls dazu führen kann, dass sich die Schraubverbindung löst und das Modul, das häufig nicht vom Anwender, gehalten wird, zu Boden fällt, wobei es zu Beschädigungen kommt.

Ferner sollte das Stroboskopmodul so mit dem Lichtanschluss koppelbar sein, dass ein an dem Stroboskopmodul angebrachtes Mikrofon auf einfache Weise in Richtung des Patienten ausgerichtet werden kann.

Ferner sollte die Kupplung so ausgestaltet sein, dass damit das Stroboskopmodul mit möglichst vielen verschiedenen auf dem Markt erhältlichen Endoskopen koppelbar ist, und zwar möglichst unabhängig vom Design des Lichtanschlusses.

Aus der WO 01/89598 A2 ist ein Führungsdraht mit einem Schaft bekannt, der eine optische Faser und eine optische Handhabe aufweist, durch die eine direkte Visualisierung bereitgestellt wird. Die optische Handhabe weist ferner einen lösbaren Sicherungsmechanismus auf, der u.a. ein Sicherungsmechanismus vom Toughy-Borst-Typ sein kann.

Es ist somit eine Aufgabe der Erfindung, eine Kupplung für ein Stroboskopmodul sowie ein Stroboskopmodul der eingangs genannten Art zu schaffen, mit dem eine einfache Ausrichtung des Stroboskopmoduls, eine sichere Verbindung von Stroboskopmodul und Endoskop und eine verbesserte Einkopplung des Lichts vom Stroboskop in den Lichtleiter durch ein Verbindung erreicht wird, die mit nur einem Handgriff verriegelt und gelöst werden kann.

Erfindungsgemäß wird die Aufgabe dadurch gelöst, dass der Lichtleiter so mit der Hülse oder dem Sockel verbunden ist, dass der Lichtleiter bei der Bewegung von der ersten Stellung in die zweite Stellung in Richtung des Lichtanschlusses verschoben wird.

Diese Maßnahme hat den Vorteil, dass der Lichtleiter beim Verbinden der Kupplung mit dem Endoskop nahe an den Lichtanschluss herangeführt wird, wodurch ein besonders effektives Einkoppeln des Lichts in den Lichtanschluss erreicht wird.

In einer bevorzugten Ausführungsform der Erfindung ist der Sockel kraftschlüssig an dem Stroboskopmodul angebracht und die Hülse ist gegenüber dem Sockel verschiebbar.

Dies hat zum einen den Vorteil, dass diese Maßnahme sowohl z.B. durch Verschrauben, Verkleben oder Formschuss des Sockels mit dem Stroboskopmodul einfach realisierbar ist. Ferner ist durch diese Maßnahme durch Einstecken des Lichtanschlusses, Verschieben der Hülse und Verriegeln gegenüber dem Sockel die Kupplung im Einhandbetrieb zu betätigen.

In einer weiteren Ausgestaltung weist die Kupplung eine Kopplungsvorrichtung auf, mit der die Hülse und der Sockel durch eine Drehbewegung zwischen der ersten und der zweiten Stellung hin- und herbewegbar sind.

Dies hat den Vorteil, dass bei gleichzeitigem Festhalten des Stroboskopmoduls die Kupplung mit zwei, sollte an der Kopplungsvorrichtung noch ein Hebel befestigt sein, sogar nur mit einem einzigen Finger erfolgen kann, was die Handhabung besonders einfach macht.

In einer weiteren Ausgestaltung der zuvor genannten Maßnahme weist die Kopplungsvorrichtung ein Innengewinde und ein Außengewinde auf, wobei das Innengewinde kraftschlüssig mit der Hülse und das Außengewinde kraftschlüssig mit dem Sockel verbunden ist.

Diese Ausgestaltung ist durch ein einfaches Eindrehen der Gewinde konstruktiv einfach und preiswert in der Herstellung.

In einer Ausgestaltung der zuvor genannten Maßnahme weisen das Innengewinde und das Außengewinde im Bereich der zweiten Stellung eine Steigung gleich Null auf.

Dadurch dass die Gewinde im Bereich der zweiten Stellung eine Steigung von Null aufweisen, ergibt sich in diesem Bereich auch eine Rückstellkraft von Null, was zu einem sicheren Verriegeln in der zweiten Stellung führt, wobei gleichzeitig dadurch, dass keine negative Steigung zu überwinden ist, die Verriegelung auch ohne übermäßigen Kraftaufwand gelöst werden kann.

In einer weiteren Ausgestaltung der Erfindung weist die Kopplungsvorrichtung eine Nut und einen Zapfen auf.

Bei dieser Ausgestaltung handelt es sich um eine konstruktiv einfache und herstellungstechnisch günstige Variante der Kopplungsvorrichtung.

In einer weiteren Ausgestaltung der zuvor genannten Maßnahme weist die Nut im Bereich der zweiten Stellung eine Steigung gleich Null auf.

Diese Maßnahme führt dazu, dass die Rückstellkraft, die auf den Zapfen wirkt, im Bereich der zweiten Stellung gleich Null ist, was zu einer sicheren Verriegelung in der zweiten Position führt, wobei gleichzeitig dadurch, dass keine negative Steigung vorliegt, die Verriegelung auch ohne übermäßigen Kraftanspruch gelöst werden kann.

In einer weiteren Ausgestaltung der Erfindung besteht die Manschette aus Kunststoff und insbesondere aus Silikon.

Kunststoffe im Allgemeinen und Silikon insbesondere sind für die Manschette vorteilhaft, da sie auf der einen Seite zu einem sicheren Reibschluss führen und gleichzeitig auch langfristig elastisch bleiben.

In einer weiteren Ausgestaltung der Erfindung ist die Manschette als Hohlzylinder ausgebildet.

Diese hat den Vorteil, dass eine solche Manschette einfach herzustellen ist. Ferner führt dies zum einem vollständigen Umfassen des Lichtanschlusses durch die Manschette und somit zu einer besonders sicheren Befestigung.

Es versteht sich, dass die vorstehend genannten und nachstehend noch zu nennenden Merkmale nicht nur in der jeweils angegebenen Kombination, sondern auch in anderen Kombinationen oder in Alleinstellung anwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: eine Kupplung eines Stroboskopmoduls mit eingeführtem Lichtanschluss in geöffneter Stellung im Teilabschnitt,
- Fig. 2: die Kupplung eines Stroboskopmoduls von Fig. 1 mit eingeführtem Lichtanschluss in geschlossener Stellung im Teilabschnitt, und
- Fig. 3: ein Stroboskopmodul im Schnitt.

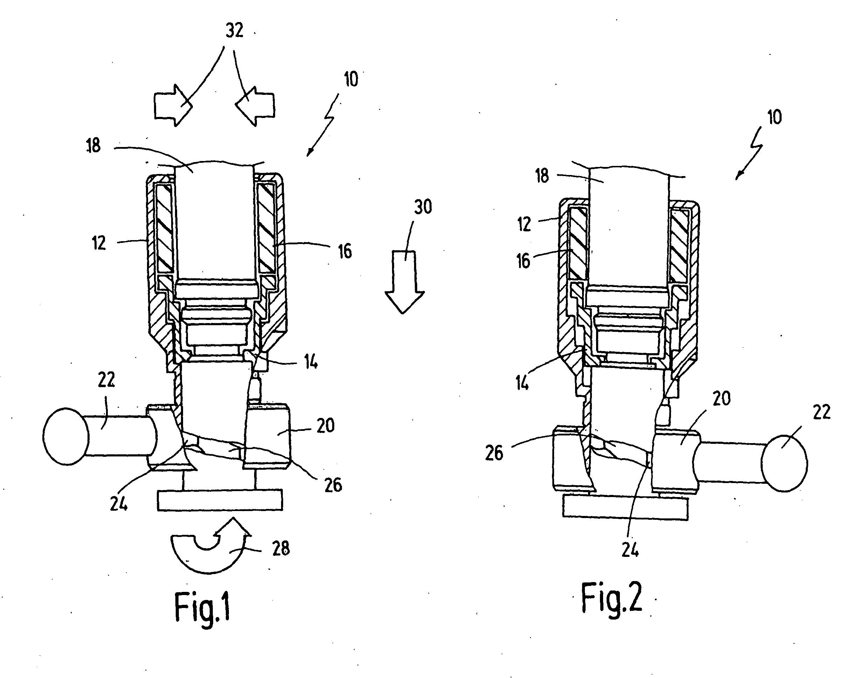

In Fig. 1 ist eine Kupplung für ein Stroboskopmodul in ihrer Gesamtheit mit der Bezugsziffer 10 bezeichnet.

Diese Kupplung 10 weist eine Hülse 12 und einen Sockel 14 auf, wobei die Hülse 12 gegenüber dem Sockel 14 verschiebbar ist. Der Sockel 14 ist in diesem Fall fest mit einem hier nicht dargestellten Stroboskopmodul verbunden.

Zwischen der Hülse 12 und dem Sockel 14 ist eine Manschette 16 aus Silikonkautschuk in Form eines Hohlzylinders angeordnet. Die Manschette 16 wird entlang ihrer äußeren Mantelfläche an der Hülse 12 umfasst, wobei die Hülse 12 an ihrem oberen Ende die obere Endfläche der Manschette 16 teilweise umfasst.

In den Innenraum der Manschette 16 und damit auch in die Hülse 12 ist ein Lichtanschluss 18 eines hier nicht dargestellten Endoskops eingeführt.

Die Kupplung 10 weist ferner eine Mutter 20 auf, die mittels eines Handgriffs 22 drehbar um den Sockel 14 gelagert ist und kraftschlüssig, jedoch gegen diese verdrehbar, mit der Hülse 12 verbunden ist.

Die Mutter 20 weist ferner einen Zapfen 24 auf, der in eine Wendelnut 26 im Sockel 14 eingreift. Diese bilden eine Kopplungsvorrichtung, mit der die lineare Bewegung der Hülse 12 gegenüber dem Sockel 14 durch eine Drehbewegung bewirkt werden kann.

Zur Befestigung des Stroboskopmoduls mit dem Lichtanschluss 18 eines Endoskops wird der Lichtanschluss 18 in die Manschette 16 eingeführt. Danach wird die Mutter 20 mittels des Handgriffs 22 in Richtung des Pfeils 28 gedreht, wobei der Zapfen 24 der Wendelnut 26 folgt. Hierdurch wird die Drehbewegung der Mutter 20 in eine Linearbewegung in Richtung des Pfeils 30 umgesetzt. Auf diese Weise werden die Hülse 12 und der Sockel 14 von der ersten Stellung in die zweite Stellung überführt.

Durch die kraftschlüssige Verbindung zwischen der Mutter 20 und der Hülse 12 wird die Hülse 12 in Richtung des Pfeils 30 nach unten bewegt. Dadurch, dass die Hülse 12 die obere Endfläche der Manschette 16 teilweise umfasst, wird die Manschette 16 von der Hülse 12 auf den Sockel 14 gepresst und verformt sich. Dabei expandiert die Manschette 16 in Richtung der beiden Pfeile 32 in Richtung ihres Innenraums wodurch der Innendurchmesser der Manschette 16 verkleinert wird und es zum Reibschluss zwischen der Manschette 16 und dem Lichtanschluss 18 kommt.

Dadurch, dass die Hülse 12 die Manschette 16 an deren äußerer Mantelfläche umfasst, wird eine Expansion der Manschette 16 nach außen wirksam verhindert. In dem Zustand nach diesen Bewegungen, der in Fig. 2 dargestellt ist, ist die Kupplung 10 durch den Reibschluss zwischen der Manschette 16 und dem Lichtanschluss 18 sowohl in Längsrichtung als auch verdrehsicher mit dem Lichtanschluss 18 verbunden. Auf diese Weise kann ein Stroboskopmodul ordnungsgemäß ausgerichtet und sicher mit einem Lichtanschluss 18 eines Endoskops verbunden werden.

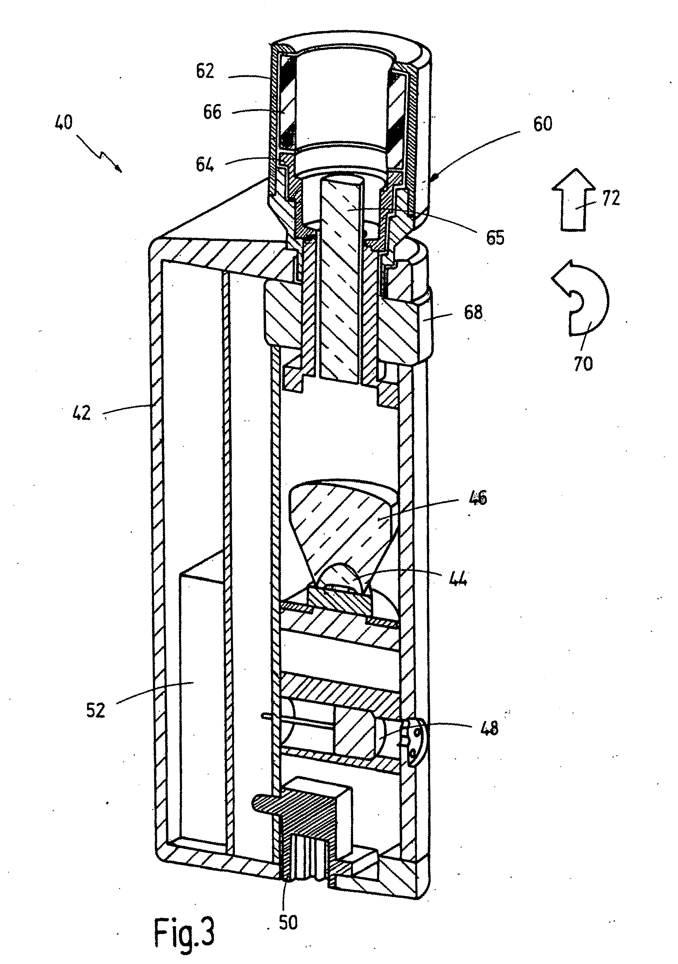

In Fig. 3 ist ein Stroboskopmodul in seiner Gesamtheit mit der Bezugsziffer 40 bezeichnet.

Das Stroboskopmodul 40 weist ein Gehäuse 42 auf, in dem eine LED 44 mit einer vorgeschalteten Fokussierlinse 46 angeordnet ist. Diese bilden die stroboskopische Lichtquelle.

Ferner ist in dem Gehäuse 42 ein Mikrofon 48 angebracht, das dazu dient, einen von einem Patienten erzeugten Ton aufzunehmen. Dieses Mikrofon 48 ist durch im Gehäuse 42 angebrachte Löcher mit der Außenwelt verbunden.

Am Boden des Beleuchtungsmoduls 40 ist ein Stecker 50 angeordnet, der dazu dienen kann, entweder einen Lithiumionenakku 52 zu laden, oder das Stroboskopmodul 40 direkt mit Strom zu versorgen.

Im Betrieb nimmt das Mikrofon 48 einen von einem Patienten erzeugten Ton auf und leitet diesen Ton an eine hier nicht dargestellte Steuereinrichtung weiter, die in Abhängigkeit von der Frequenz dieses Tons die LED 44 ansteuert, die wahlweise Dauerlicht oder gepulstes Licht abgibt, wobei die Frequenz des gepulsten Lichtes von der Frequenz des vom Patienten abgegebenen Tons abhängt. Die hier nicht dargestellte Steuereinheit kann ferner die Frequenz des abgegebenen Tons auf einer hier ebenfalls nicht dargestellten Anzeige anzeigen.

Am oberen Abschnitt des Gehäuses 40 ist oberhalb der LED 44 eine Kupplung 60 angeordnet.

Diese Kupplung 60 weist eine Hülse 62 und einen Sockel 64 auf, wobei innerhalb des Sockels 64 ein Lichtleiter in Form eines Quarzstabs 65 angeordnet ist, der dazu dient, das von der LED 44 und der Fokussierlinse 46 abgegebene Licht in einen Lichtleiter eines Endoskops hineinzufokussieren. Zwischen der Hülse 62 und dem Sockel 64 ist wiederum eine Manschette 66 in Form eines Hohlzylinders aus einem elastischen Kunststoff angeordnet.

Die Kupplung 60 weist ferner eine Mutter 68 auf, die über ein hier nicht dargestelltes Innengewinde mit einem hier nicht dargestellten Außengewinde des Sockels 64 verbunden ist.

Wird nun die Mutter 68 in Richtung des Pfeils 70 gedreht, greift das Innengewinde der Mutter 68 in das Außengewinde des Sockels 64 ein und verschiebt diesen in Richtung des Pfeils 72 nach oben. Hierdurch wird die Manschette 66 gegen die Hülse 62 gepresst, die die Manschette an deren oberen Ende umfasst, und verformt. Am Ende dieser Drehbewegung weisen sowohl das Innengewinde der Mutter 68 wie auch das Außengewinde des Sockels 64 eine Steigung gleich Null auf, wodurch der Sockel 64 in dieser Position verriegelt wird. Durch die Deformation der Manschette 66 kann es, wenn in die Kupplung 60 ein Lichtanschluss eines Endoskops eingeführt ist, zwischen dem Lichtanschluss und der Manschette 66 zum Reibschluss kommen, wodurch das Stroboskopmodul 40 sicher mit dem Lichtanschluss und damit dem Endoskop verbunden ist.

Dadurch dass der Quarzstab 65 mit dem Sockel 64 verbunden ist wird dieser ebenfalls in Richtung des Pfeils 72 nach oben, also in Richtung des hier nicht dargestellten Lichtanschlusses verschoben. In dem mit dem Endoskop verbundenen Zustand ist der Quarzstab dabei so angeordnet, dass dieser in einem Abstand von 0,7 mm vor eine Stutzen des Lichtanschlusses zu liegen kommt, wodurch ein besonders effektives Einkoppeln von Licht aus dem Stroboskopmodul in den Lichtanschluss sichergestellt ist.

## Patentansprüche

1. Kupplung für ein Stroboskopmodul, die eine Hülse (12; 62) und einen gegen die Hülse (12; 62) verschiebbaren Sockel (14; 64) aufweist, wobei zwischen der Hülse (12; 62) und dem Sockel (14; 64) eine elastisch verformbare Manschette (16; 66) angeordnet ist, in die in einer ersten Stellung ein Lichtanschluss (18) eines Endoskops einführbar ist, wobei die Hülse (12; 62) und der Sockel (14; 64) in einer zweiten Stellung die Manschette (1.6; 66) komprimieren, so dass sich der Innendurchmesser der Manschette (16; 66) verkleinert, wobei die Hülse (12; 62) und der Sockel (14; 64) in der zweiten Stellung verriegelbar sind, und wobei die Kupplung (10; 60) ferner einen Lichtleiter aufweist, der beim Anschluss des Stroboskopmoduls (40) an ein Endoskop Licht aus dem Stroboskopmodul (40) in den Lichtanschlusses (18) des Endoskops einkoppelt, **dadurch gekennzeichnet, dass** der Lichtleiter so mit der Hülse (12; 62) oder dem Sockel (14; 64) verbunden ist, dass der Lichtleiter bei der Bewegung von der ersten Stellung in die zweite Stellung in Richtung des Lichtanschlusses (18) verschoben wird.

2. Kupplung nach Anspruch 1, **dadurch gekennzeichnet, dass** der Sockel (14; 64) kraftschlüssig mit dem Stroboskopmodul (40) verbunden ist und die Hülse (12; 62) gegenüber dem Sockel (14; 64) verschiebbar ist.

3. Kupplung nach Anspruch 1 oder 2, **dadurch gekennzeichnet, dass** die Kupplung (10; 60) eine Kopplungsvorrichtung aufweist, mit der die Hülse (12; 62) und der Sockel (14; 64) durch eine Drehbewegung zwischen der ersten Stellung und der zweiten Stellung hin und her bewegbar sind.

4. Kupplung nach Anspruch 3, **dadurch gekennzeichnet, dass** die Kopplungsvorrichtung ein Innengewinde und ein Außengewinde aufweist, wobei das Innengewinde kraftschlüssig mit der Hülse (12; 62) und das Außengewinde kraftschlüssig mit dem Sockel (14; 64) verbunden ist.

5. Kupplung nach Anspruch 4, **dadurch gekennzeichnet, dass** das Innengewinde und das Außengewinde im Bereich der zweiten Stellung eine Steigung gleich Null aufweisen.

6. Kupplung nach Anspruch 3, **dadurch gekennzeichnet, dass** die Kopplungsvorrichtung eine Nut (26) und einen Zapfen (24) aufweist.

7. Kupplung nach Anspruch 6, **dadurch gekennzeichnet, dass** die Nut (26) im Bereich der zweiten Stellung eine Steigung gleich Null aufweist.

8. Kupplung nach einem der Ansprüche 1 bis 7, **dadurch gekennzeichnet, dass** die Manschette (16; 66) aus Kunststoff besteht.

9. Kupplung nach Anspruch 8, **dadurch gekennzeichnet, dass** die Manschette (16; 66) aus Silikon besteht.

10. Kupplung nach einem der Ansprüche 1 bis 9, **dadurch gekennzeichnet, dass** die Manschette (16; 66) als Hohlzylinder ausgebildet ist.

11. Stroboskopmodul mit einer Kupplung (10; 60) mit der das Stroboskopmodul (40) an einen Lichtanschluss (18) eines Endoskops anschließbar ist, **gekennzeichnet durch** eine Kupplung nach einem der Ansprüche 1 bis 10.

## Claims

1. Coupling for a stroboscope module comprising a sleeve (12; 62), and a base (14; 64) that is displaceable relative to the sleeve (12; 62), whereby an elastically deformable cuff (16; 66) is arranged between the sleeve (12; 62) and the base (14; 64), into which cuff (16; 66) a light attachment (18) of an endoscope can be inserted in a first position, whereby the sleeve (12; 62) and the base (14; 64) compress the cuff (16; 66) in a second position such that the internal diameter of the cuff (16; 66) is reduced, whereby the sleeve (12; 62) and the base (14; 64) can be locked in the second position, and whereby the coupling (10; 60) moreover comprises a light guide which, when the stroboscope module (40) is connected to an endoscope, couples light from the stroboscope module (40) into the light attachment (18) of the endoscope, **characterized in that** the light guide is connected to the sleeve (12; 62) or to the base (14; 64) such that the light guide, during the movement from the first position to the second position, is displaced in the direction of the light attachment (18).

2. Coupling according to Claim 1, **characterized in that** the base (14; 64) is connected with a force fit to the stroboscope module (40), and the sleeve (12; 62) is displaceable relative to the base (14; 64).

3. Coupling according to Claim 1 or 2, **characterized in that** the coupling (10; 60) comprises a coupling device with which the sleeve (12; 62) and the base (14; 64) can be moved to and fro between the first position and the second position by a rotational movement.

4. Coupling according to Claim 3, **characterized in that** the coupling device comprises an inner thread and an outer thread, whereby the inner thread is connected with a force fit to the sleeve (12; 62) and the outer thread is connected with a force fit to the base (14; 64).

5. Coupling according to Claim 4, **characterized in that** the inner thread and the outer thread have a pitch equal to zero in the region of the second position.

6. Coupling according to Claim 3, **characterized in that** the coupling device comprises a groove (26) and a peg (24).

7. Coupling according to Claim 6, **characterized in that** the groove (26) has a pitch equal to zero in the region of the second position.

8. Coupling according to one of Claims 1 to 7, **characterized in that** the cuff (16; 66) is made of plastic.

9. Coupling according to Claim 8, **characterized in that** the cuff (16; 66) is made of silicone.

10. Coupling according to one of Claims 1 to 9, **characterized in that** the cuff (16; 66) is designed as a hollow cylinder.

11. Stroboscope module having a coupling (10; 60) with which the stroboscope module (40) can be connected to a light connection (18) of an endoscope, **characterized by** a coupling according to one of claims 1 to 10.

## Revendications

1. Accouplement pour un module de stroboscope, qui présente un manchon (12 ; 62) et un socle (14; 64) déplaçable par rapport au manchon (12 ; 62), une manchette déformable élastiquement (16 ; 66) étant disposée entre le manchon (12 ; 62) et le socle (14 ; 64), dans laquelle, dans une première position, peut être introduite une connexion lumineuse (18) d'un endoscope, le manchon (12 ; 62) et le socle (14 ; 64) comprimant dans une deuxième position la manchette (16 ; 66) de façon que le diamètre intérieur de la manchette (16 ; 66) soit rétréci, le manchon (12 ; 62) et le socle (14 ; 64) étant verrouillables dans la deuxième position, et l'accouplement (10 ; 60) présentant en outre un guide de lumière qui, lors de la connexion du module de stroboscope (40) à un endoscope, injecte de la lumière du module de stroboscope (40) dans la connexion lumineuse (18) de l'endoscope, **caractérisé en ce que** le guide de lumière est relié au manchon (12 ; 62) ou au socle (14 ; 64) de façon que le guide de lumière, lors du déplacement de la première position dans la deuxième position, soit déplacé en direction de la connexion lumineuse (18).

2. Accouplement selon la revendication 1, **caractérisé en ce que** le socle (14 ; 64) est relié par conjugaison de forces au module de stroboscope (40) et le manchon (12 ; 62) est déplaçable par rapport au socle (14 ; 64).

3. Accouplement selon la revendication 1 ou 2, **caractérisé en ce que** l'accouplement (10 ; 60) présente un dispositif de couplage avec lequel le manchon (12 ; 62) et le socle (14 ; 64) sont déplaçables alternativement entre la première position et la deuxième position par un mouvement de rotation.

4. Accouplement selon la revendication 3, **caractérisé en ce que** le dispositif de couplage présente un filetage intérieur et un filetage extérieur, le filetage intérieur étant relié par conjugaison de forces au manchon (12 ; 62) et le filetage extérieur par conjugaison de forces au socle (14 ; 64).

5. Accouplement selon la revendication 4, **caractérisé en ce que** le filetage intérieur et le filetage extérieur présentent un pas égal à zéro dans la région de la deuxième position.

6. Accouplement selon la revendication 3, **caractérisé en ce que** le dispositif de couplage présente une rainure (26) et un tenon (24).

7. Accouplement selon la revendication 6, **caractérisé en ce que** la rainure (26) présente un pas égal à zéro dans la région de la deuxième position.

8. Accouplement selon l'une des revendications 1 à 7, **caractérisé en ce que** la manchette (16 ; 66) est en matière plastique.

9. Accouplement selon la revendication 8, **caractérisé en ce que** la manchette (16 ; 66) est en silicone.

10. Accouplement selon l'une des revendications 1 à 9, **caractérisé en ce que** la manchette (16 ; 66) est réalisée sous la forme d'un cylindre creux.

11. Module de stroboscope avec un accouplement (10 ; 60) avec lequel le module de stroboscope (40) peut être connecté à une connexion lumineuse (18) d'un endoscope, **caractérisé par** un accouplement selon l'une des revendications 1 à 10.
